# NEUE EUROPÄISCHE PATENTSCHRIFT

(11) **EP 0 399 549 B2**
(45) Veröffentlichungstag und Bekanntmachung des Hinweises auf die Entscheidung über den Einspruch: **26.05.1999**
(45) Hinweis auf die Patenterteilung: 18.11.1993
(21) Anmeldenummer: 90109963.0
(22) Anmeldetag: 25.05.1990
(51) Int. Cl.: A61M 1/14, A61M 1/28

(54) **Zweikammerbeutelsystem zur Herstellung einer für die CAPD einsetzbaren Dialysierflüssigkeit**
CAPD-bag system with two chambers
Système de poche avec deux chambres pour dialyse péritonéale

(30) Priorität: 26.05.1989 DE 3917251
(43) Veröffentlichungstag der Anmeldung: 28.11.1990
(73) Patentinhaber: Fresenius AG, 61350 Bad Homburg (DE)
(72) Erfinder: Reinhardt, Bertold, Dr., D-6370 Oberursel (DE); Bartz, Volker, Dip.-Betriebswirt, D-6307 Linden (DE)
(74) Vertreter: Luderschmidt, Schüler & Partner GbR

(56) Entgegenhaltungen:
- EP-A- 0 022 922
- EP-A- 0 076 658
- EP-A- 0 086 553
- EP-A- 0 161 471
- EP-A- 0 277 868
- The International Journal of Artificial Organs, Vol. 8, No. 3, Seiten 121 - 124, T. S. Ing et al, "Biocarbonate-buffered peritoneal dialysis", 1985
- U. Graefe et al., "Less Dialysis-Induced Morbidity and Vascular Instability with Bicarbonate in Dialysate", Ann. Intern. Med. 88, Seite 332, 1978
- Klein et al., "Calcium Carbonate Precipitation in Bicarbonate Hemodialysis", Artificial Organs, Vol. 10, No. 3, 1986
- Siggaard Andersen O., "The first dissociation exponent of carbonic acid as a function of pH", Scand. J. Clin & Lab Inv. Seite 587 - 579, 14, 1962
- Gorman Hills, "Acid-base Balance", The Williams & Wilkins Company, Baltimore 1973
- Siggaard Anderson O., "The Acid-base Status of the Blood", 4th Ed., Munksgaard 1976, Seite 30 - 31

## Beschreibung

Die Erfindung betrifft Zweikammerbeutelsystem zur Herstellung einer für die CAPD (kontinuierliche ambulante Peritonealdialyse) einsetzbaren Dialysierflüssigkeit, die folgende Zusammensetzung in mval/l aufweist:
Ca²⁺ = 0,5 - 5
Mg²⁺ = 0 - 3
Cl⁻ = 90,5 - 121
Na⁺ = 128 - 145
K⁺ = 0 - 4
HCO₃ = 25 - 40

Neben der Entfernung von Stoffwechselprodukten besteht eine der wichtigsten Aufgaben jeder Nierenersatztherapie, wie Hämodialyse, Hämofiltration, Hämodiafiltration, CAVH, CAVHD und der Peritonealdialyse (CAPD), in der Korrektur der metabolischen Acidose. Aus diesem Grunde enthalten die in jedem dieser Verfahren verwendeten Dialysierflüssigkeiten einen Puffer.

In der Hämodialyse wie auch in der CAPD wurde zunächst Bicarbonat in Form des Natriumbicarbonats als- Puffer verwendet, aber nach ersten Versuchen durch andere Puffer, wie Lactat (üblicherweise in der CAPD) oder Acetat (üblicherweise in der Hämodialyse) ersetzt. Neben der technisch damals noch nicht beherrschbaren Stabilität der Dialysierflüssigkeiten warinsbesondere die Ausfällung von Calciumcarbonat in die sen Lösungen der Grund für den Austausch der Puffersubstanzen. Da zur Therapierung von urämischen Patienten Calciumkonzentrationen von etwa 2 mmol/l und Bicarbonatkonzentrationen bis zu 42 mmol/l verwendet bzw. benötigt werden, kommt es, begünstigt durch die Art der Verwendung bzw. des Einsatzes dieser Lösungen, zu einem Überschreiten des Löslichkeitsproduktes von Calcium und Carbonat und folglich zur Präzipitation von Calciumcarbonat in der Lösung. Verstärktwird das Problem der Calciumcarbonatausfällung bei CAPD-Lösungen dadurch, daß sie aus Sterilitätsgründen bei etwa 120°C autoklaviert werden müssen.

In der Hämodialyse und den mit ihr verwandten Verfahren werden daher schon seit einigen Jahren Bicarbonat als Puffer enthaltende Dialysierflüssigkeiten in der Weise hergestellt, daß einerseits ein basisches Bi-carbonatkonzentrat und andererseits ein Calciumionen enthaltendes saures Elektrolytkonzentrat in getrennten Behältern gehalten werden. Diese Behälter werden an die Dialysemaschine angeschlossen, die erst unmittelbar vor dem Gebrauch die beiden Konzentrate zusammen mit Wasser zu der endgültigen Dialysierflüssigkeit mischt Selbst bei dieser Art der Herstellung und des unmittelbaren Einsatzes erfolgt noch eine Calciumcarbonatausfällung in den Dialysemaschinen, was durchaus zu Störungen des Dialysebetriebes führen kann. Infolgedessen werden zur Vermeidung von Langzeitkomplikationen sämtliche Rohrleitungen der Dialysemaschine in regelmäßigen Abständen mit Saure, wie Essigsäure oder anderen verdünnten Säuren, gespült, um das Calciumcarbonat zu entfernen.

Aus dem Stand der Technik sind eine Reihe von Patentschriften bekannt, die sich mit der Herstellung von Bicarbonat enthaltenden Diaiysierflüssigkeiten für die Blutreinigung beschäftigen. In sämtlichen Druckschriften ist jeweils der Einsatz eines sauren Konzentrats und eines basischen Bicarbonatkonzentrates vorgeschlagen worden, wobei sich die pH-Werte beider Konzentrate jeweils durch die pH-Werte der eingesetzten dissoziierbaren Salze (Calciumchlorid, Natriumbicarbonat oder Natriumcarbonat) zwangsläufig ergeben. Dabei kann zusätzliche Säure zum sauren Konzentrat hinzugegeben werden, um den Säuregrad anzuheben, d.h. den gemessenen pH-Wert zu senken, um nach Vermischen mit dem basischen Konzentrat den physiologischen pH-Wert von etwa 7,3 zu erhalten.

Vorrichtungen zur Herstellung einer Bicarbonat enthaltenden Dialysierflüssigkeit, Konzentrate und die Dialysierflüssigkeiten selbst sind beispielweise in der DE-A-31 46 425 = WO81/03180 (D1), der EP-A-022 922 und der EP-A-086 553 sowie den in der letztgenannten Druckschrift zitierten Patentschriften beschrieben. Sämtlichen Patentschriften ist gemein, daß sie keinerlei Korrektur des pH-Wertes für das Bicarbonat enthaltende Konzentrat vorschlagen, um hierdurch die Calciumcarbonatausfällung nach dem Vermischen mit dem calciumhaltigen Konzentrat zu beschränken bzw. sogar aufzuheben. Dies ist insofern erklärlich, als durch Zugabe von Säure bekanntlich das Bicarbonat-CO₂-Gleichgewicht auf die Seite des CO₂ verschoben, d.h. gasförmiges CO₂ freigesetzt wird. Dies macht besondere Maßnahmen zur Stabilisierung der Behälter notwendig, da ansonsten CO₂ entweicht und hierdurch wieder der pH-Wert angehoben, d.h. in den basischen Bereich verschoben wird.

Insofern wurde aus chemischen Gründen das bereits saure Calciumionen enthaltende Konzentrat weiter mit Säure angesäuert, um nach dem Vermischen mit dem Bicarbonat-Konzentrat die gewünschte Zusammensetzung der Dialyseirflüssigkeit zu erhalten.

Ähnlich wie bei der Hämodialyse wurde nach einem Vorschlag von Feriani und La Greca in der CAPD zur Wiedereinführung des Bicarbonats anstelle des weniger physiologischen Lactats als Puffer das Bicarbonatkonzentrat von dem Calcium enthaltenden Elektrolytkonzentrat in einem Zweikammerbeutel getrennt, der in der EP-A-161 471 beschrieben ist. Weitere Veröffentlichungen dieser Autoren finden sich in int.JArt.Organs (1985) S.57-58 und in der Monographie "PERITONEAL DIALYSIS"-Proc.2nd Int.Course (1986), S.143-148.

Mit der in diesen Druckschriften beschriebenen Anordnung, in der die beiden Konzentrate in zwei miteinander verbindbaren Kammern gehalten werden, läßt sich ohne Schwierigkeiten eine Autoklavierung durchführen. Dennoch kann eine Calciumcarbonatausfällung nach dem Mischen oder während der Behandlung im Peritonealraum nicht ausgeschlossen werden, da unmittelbar nach dem Vermischen in relativ kurzen Zeiten (höchstens zwei Stunden) Calciumcarbonat ausfällt, was bei der Durchführung der Peritonealdialyse nicht akzeptiert werden kann.

Zur Vermeidung der Calciumcarbonatausfällung verwendeten die Autoren daherverdünnte Lösungen von Natriumbicarbonat, d.h. einem Bicarbonatgehalt unter 30 mmol/l und einer Calciumkonzentration von 1,5 mmol/l in der fertigen Flüssigkeit. Abgesehen davon, daß hierdurch die Ausfällungsgefahr immer noch nicht beseitigt war, reichten diese Bicarbonatkonzentrationen nicht aus, um die Acidose der Patienten ausreichend zu korrigieren. So stiegen die Bicarbonatplasmaspiegel der Patienten nicht über 22 mmol/l an und lagen meist deutlich darunter, wobei auf einen normalen Bicarbonatplasmaspiegel von etwa 25 mmol/l bezogen wurde.

Der Erfindung liegt daher die Aufgabe zugrunde, ein Zweikammerbeutelsystem zur Herstellung einer bicarbonathaltigen Dialysierflüssigkeit zur Verfügung zu stellen, bei dem nicht die Gefahr besteht, daß beim Mischen oder innerhalb der Dialysebehandlungsdauer Calciumcarbonat ausfällt.

Die Lösung der Aufgabe erfolgt dadurch, daß das Natriumbicarbonatkonzentrat in einer Kammer so viel zugesetzte physiologisch verträgliche Säure aufweist, daß der pH-Wert des Konzentrats unter 7,6 bei Raumtemperatur liegt.

Vorzugsweise stellt man den pH-Wert des Natriumbicarbonatkonzentrates mit der Säure auf einen pH-Wert von 7,2 - 7,4, insbesondere etwa 7,3 - 7,35 bei Raumtemperatur ein.

Die Einstellung des pH-Werts auf 7,4 hat den Vorteil, daß der Carbonatgehalt in diesem Konzentrat etwa tausendmal niedriger ist als in Bicarbonatkonzentraten mit einem pH-Wert um 8. Letztere Konzentrate werden nach dem Stand der Technik mit einem solchen pH-Wert und darüber (bis 8,8) in den Handel gebracht, so daß die Bildung von Mikrokristallen bzw. Präkeimen von Calciumcarbonat in den bekannten Konzentraten begünstigt wird. Es kam daher bei den bekannten Konzentraten zu einer Ausfällung von Calciumcarbonat, selbst wenn zu den von Feriani et al vorgeschlagenen Bicarbonatkonzentrationen unter 30 mmol/l in der fertigen Dialysierflüssigkeit weiteres Bicarbonat zugeführt wurde, um die Bicarbonatkonzentration über 32 mmol/l anzuheben.

Erfindungsgemäß wurde zusätzlich festgestellt, daß es beim Mischen der bekannten Konzentrate zu lokalen Überschreitungen des Löslichkeitsprodukts von Calciumcarbonat für solche übersättigten Lösungen kommt, vor allem, wenn beim Durchmischen das Calcium im alkalischen Bereich mit dem dort vorhandenen Carbonat zusammentrifft, obwohl das Säurekonzentrat, also die Calcium enthaltende Elektrolytlösung, in der Regel so angesäuert ist, daß das Gemisch aus Elektrolytlösung und Bicarbonatkonzentrat nach dem Vermischen einen pH-Wert im physiologischen Bereich von 7,2 - 7,4 erreicht. Bei dieser Vorgehensweise hat es die Fachwelt unberücksichtigt gelassen, daß es bei der Mischung der beiden Konzentrate zu kurzfristigen signifikanten Überschreitungen des Löslichkeitsproduktes von Caiciumcarbonat im alkalischen Bereich kommt. Diese kurze Zeit ist jedoch ausreichend für die Bildung von üblicherweise mit dem Auge noch nicht erkennbaren Calciumcarbonatkeimen, die je nach den physikalischen Umständen zu einer verzögerten Calciumcarbonatausfällung führen. Insofern ist also die bekannte Lösung als metastabil zu betrachten, selbst wenn sie unmittelbar nach dem Vermischen noch klar erscheint, d.h. die Stabilität einer solchen Lösung ist hinsichtlich des Nichtauftretens einer Calciumcarbonatausfällung erheblich vermindert. Dies läßt sich bereits nach wenigen Minuten, manchmal auch nach einigen Stunden, feststellen, wenn es zu der gefürchteten Calciumcarbonatausfällung kommt.

Dabei sind Zeitpunkt und Ausmaß der Ausfällung abhängig von der Konzentration beider Komponenten (Calcium und Carbonat), dem "lokalen" pH-Wert, der lonenstärke der Lösung sowie der Temperatur und dem Druck.

Aufgrund des eingestellten pH-Wertes bei der erfindungsgemäßen Lösung kommt es jedoch nicht zum Überschreiten des Löslichkeitsprodukts bei der Vermischung der beiden Konzentrate und somit nicht zu einer Calciumcarbonatausfällung, sofern das zwangsläufig aus physikalisch-chemischen Gründen vorliegende CO₂ in der Lösung gehalten wird. Nur wenn man die Lösung offen stehen läßt d.h. das CO₂ entweichen kann, kommt es durch die hierdurch zwangsläufige Erhöhung des pH-Wertes auf 8 und darüber, wiederum zu einem Überschreiten des Löslichkeitsproduktes nach Stunden und damit zu einer beginnenden Ausfällung von Calciumcarbonat. Dies hat jedoch für die Dialyse, insbesondere die Peritonealdialyse, keine Bedeutung, da bei der Hämodialyse die Diaysierflüssigkeit längst den Dialysefilter durchlaufen hat und bei der Peritonealdialyse längst sich das physiologische Gleichgewicht innerhalb des Peritonealraums eingestellt hat.

Es muß jedoch festgestellt werden, daß es sich bei den hier beschriebenen Lösungen um hochkonzentrierte Lösungen handelt, deren chemisches Verhalten, insbesondere das Ausfällungsverhalten, nicht vorausgesagt werden kann. Insofern sind die vorstehenden Erläuterungen im Lichte der Erfindung zu sehen, die darin besteht, daß der pH-Wert der Bicarbonatkonzentrates zunächst unter 7,6 abgesenkt wird, und die so eingestellte Konzentratlösung anschließend mitdem sauren, Calciumlonen enthaltenden Konzentrat vermischt wird.

Wie festgestellt, wird bei der Vermischung mit dem erfindungsgemäßen Konzentrat eine Mikrokristallisation bzw. die Bildung von Präkeimen von Calciumcarbonat vermieden. In so hergestellten, übersättigten Lösungen ist die Einstellung der Gleichgewichte zwischen Calcium und den für eine Kompiexbildung mit Calcium in Frage kommenden Reaktionspartnern sowie den bereits zwischen den beiden Partnern gebildeten Komplexen gegenüber idealen Lösungen erheblich verzögert. Soweit ersichtlich, kommt es selbst bei einer Überschreitung des Löslichkeitsproduktes erst nach Stunden zu einer Calciumcarbonatausfällung. Insofern sind nach dem erfindungsgemäßen Verfahren hergestellte Lösungen eher stabil noch bis zu Bicarbonatkonzentrationen von etwa 60 mmol/l und bis zu Calciumkonzentrationen von etwa 5 mmol/l.

Die für die Anwendung solcher Lösungen in der CAPD notwendige Stabilität von 12 Std. (dwelltime over night) wird bei der für den Ausgleich der Acidose notwendigen Bicarbonatkonzentration um das Doppelte überschritten. Erste Untersuchungen an Tier und Mensch haben dies bewiesen.

Die erfindungsgemäßen Konzentrate, also das saure Konzentrat (A) und das basische Bicarbonatkonzentrat (B) können auf die übliche Weise, also gemäß dem Stand der Technik, wie er in der Einleitung beschrieben ist, hergestellt werden. Erfindungswesentlich ist es dabei, daß das Bicarbonatkonzentrat durch Zugabe von physiologisch verträglicher Säure auf einen pH-Wert von höchstens 7,6 eingestellt wird.

Einsetzbare Säuren sind beispielsweise Salzsäure oder organische verstoffwechselbare Säuren, wie Essigsäure oder Milchsäure, wobei allerdings festzustellen ist, daß die letztgenannten Säuren weniger bevorzugt sind, da die Fachwelt von der Acetat/Lactat-Lösung wegkommen will. Darüber hinaus kann natürlich auch das "Anhydrid" der Kohlensäure, also CO₂, soweit zugefügt werden, daß der pH-Wert der Dialysierflüssigkeit unter 7,6 gesenkt wird. Hieraus ist bereits ersichtlich, daß das fertige Konzentrat durch eine geeignete Wahl von Behältern davor geschützt werden muß, daß das in freien Gasblasen oder gelöster Form vorliegende CO₂ aus dem Konzentrat sich herauslöst und der pH-Wert wieder ansteigt.

Ein einsetzbares wäßriges Natriumbicarbonat enthaltendes Konzentrat, das mit einem sauren, Calciumcarbonat enthaltenden Konzentrat vermischt wird, kann folgende Zusammensetzung aufweisen: 72 mmol/l NaHCO₃, wobei der pH-Wert mit Hilfe von HCl auf 7,35 - 7,4 bei Raumtemperatur (25°C) eingestellt worden ist.

Dieses Konzentrat kann auf die übliche Weise sterilisiert werden, beispielsweise durch Autoklavierung bei 121°C oder aber durch Sterilisation durch ein Sterilfilter (mittlere Porengröße 0,2 µm), wobei der Filtrationsdruck nicht > 1 bar sein soll.

Dieses basische Konzentrat (B) kann miteinem sauren wäßrigen Konzentrat (A), dasfolgende Zusammensetzung aufweist:
- 196 mmol/l: NaCl
- 3,6 mmol/l: CaCl₂
- 1 mmol/l: MgCl₂
im Verhältnis von 1 : 1 vermischt werden.

Dieses Konzentrat (A) wird in gleicher Weise sterilisiert und im Bedarfsfall dann mit dem Konzentrat (B) unter sterilen Bedingungen vermischt, beispielsweise unter Zuhilfenahme des Doppelkammerbeutels, der in der EP-A-161 471 beschrieben ist.

Als Material für solche Kunststoffbeutel werden die üblichen polymeren Laminate eingesetzt. Bei Kunststoffmaterialien sind jedoch deren Gasdurchlässigkeiten zu beachten. Insbesondere soll der geschlossene Beutel aus dem bicarbonathaltigen Bereich während einer etwa halbjährlichen Lagerung nicht mehr als 5% des urprünglichen CO₂-Gehalts verlieren, d.h. der Beutel soll im wesentlichen für Gase, insbesondere CO₂ undurchlässig sein. Für diese Zwecke weist daher ein derartiges Laminat eine Gassperrschicht, insbesondere eine Aluminiumschicht auf. Diese Anforderungen lassen sich auch durch Parameterwerte ausdrücken. So sollen derartige Beutelmaterialien eine Wasserdampfdurchlässigkeit von weniger als lg/m²/Tag/bar bei 20°C, gemessen nach DIN 53122 und eine CO₂-Durchlässigkeit von weniger als lcm³/100µm/m²/Tag/bar bei 20°C, gemessen nach DIN 53380 aufweisen. In jedem Fall sollte der pH-Wert des Konzentrates innerhalb einer Bandbreite von höchstens 0,15 Einheiten zwischen den Anfangs- und Endwerten schwanken.

Andererseits können auch voneinandergetrennte Gefäße (Beutel, Flaschen), die somit mehrere Kammern aufweisen, eingesetztwerden. Diese Gefäße werden zum Mischen derjeweiligen Lösungen miteinander durch ein geeignetes Verbindungssystem (Schlauchsystem) verbunden.

Des weiteren werden die jeweiligen Konzentrate vorzugsweise in einem Verhältnis von etwa 3:1 bis 1:3, insbesondere etwa 1:1, miteinander vermischt Die Mengen der Elektrolyte bzw. des osmotischen Agens sind entsprechend dem gewählten Verdünnungsverhältnis und dereinzustellenden Endkonzentrationen vorzuwählen.

Andererseits können derartige Konzentrate natürlich auch unmittelbar in der Dialysemaschine zur Herstellung einer Dialysierflüssigkeit für die Hämodialyse gemischt werden.

Die erhaltene Lösung weist dann 134 mmol/l Natriumionen, 1,8 mmol/l Calciumionen, 0,5 mmol/l Magnesiumionen sowie ca. 34 mmol/l Natriumhydrogencarbonat (Rest CO₂ und Carbonat-Ionen) sowie Rest Chloridionen auf.

Im Bedarfsfall kann natürlich die Hydrogencarbonatkonzentration in der endgültigen Dialysierflüssigkeit entsprechend den Bedürfnissen der Patienten gewählt werden, was eine weitere unabhängige bevorzugte Ausführungsform der Erfindung ist. So lassen sich aus den Blutwerten eines urämischen Patienten das Gesamt-CO₂, also tCO₂, der Bicarbonatgehalt des Blutes und daraus der Gesamt-Bicarbonatgehalt des Patienten ermitteln und berechnen. Hieraus kann die dem Patienten bei einer Behandlung zur Verfügung zu stellende Hydrogencarbonat-Menge individuell errechnet und durch eine entsprechende Wahl einer bestimmten Lösung zur Verfügung gestellt werden. Insofern ist es mit dem Einsatz einer erfindungsgemäßen Lösung möglich, Patienten von ihrer Acidose dadurch zu befreien, daß der Bicarbonat-Pool während einer Dialysebehandlung stets aufgefüllt wird, so daß sich keinerlei Acidose-Zustände mehr ausbilden können.

Darüber hinaus hat die Einführung einer Bicarbonatdialysierflüssigkeit mit physiologischem pH-Wert in den Peritonealraum den Vorteil, daß nicht die natürliche Immunabwehr im Peritonealraum beseitigt wird, sondern sich vielmehr entfalten kann. Neuere Erkenntnisse haben nämlich gezeigt, daß die derzeit üblichen CAPD-Dialysierflüssigkeiten mit einem pH-Wert von 5,1 - 5,4 praktisch die gesamte Immunabwehr von Makrophagen im Peritonealraum lahmlegen, somit also stets die Gefahr der Entstehung einer Peritonitis durch Einschleppen von Keimen entsteht. Dies kann durch die Zurverfügungstellung einer Dialysierflüssigkeit mit physiologischem pH wirksam verhindert werden.

Wie bereits festgestellt, können Dialysierflüssigkeiten unterschiedlichen Bicarbonatgehalts aus entsprechend formulierten Konzentraten hergestellt werden, wobei üblicherweisevon einem Bicarbonatgehalt von wenigstens 20 mmol/l ausgegangen wird. Vorzugsweise sollte der Bicarbonatgehalt in derfertigen Flüssigkeit zwischen 25 und 40 mmol/l liegen. Es versteht sich von selbst, daß die tatsächlich eingewogene Bicarbonat-Menge etwas höher liegt, da - wie vorstehend festgestellt - das Bicarbonat mit CO₂ im Gleichgewicht liegt und sich bei einer Erniedrigung des pH-Wertsvon ursprünglich ca. 8-8,8 auf 7,3 - 7,4 CO₂ aus Hydrogencarbonat bildet. Diese CO₂-Menge hängt natürlich ab vom pH-Wert und liegt üblicherweise bei 5 - 10% des ursprünglich eingewogenen Hydrogencarbonats, so daß die einzuwiegende Hydrcgencarbonatmenge entsprechend zu korrigieren ist.

Anstelle von zwei Konzentraten (A) und (B) können natürlich auch drei Lösungen in Form des sauren Konzentrates (A) und einer Natriumcarbonatlösung (B1) und einer Säurelösung (B2) eingesetztwerden. Dabei werden die beiden Lösungen (B1) und (B2) zuerst gemischt, wobei (B2) einen derartigen Säuregrad aufweist, daß die endgültige Lösung einen pH-Wert von höchstens 7,6 besitzt, wie dies vorstehend erläutert ist. Diese Hydrcgencarbonatlösung entspricht dann dem vorstehend beschriebenen Konzentrat (B), das sich anschließend mit dem Konzentrat (A) vermischen läßt.

Fertige Dialysierflüssigkeiten können folgende Zusammensetzung in mval/l aufweisen:
- Ca²⁺ = 0,5 - 5: bevorzugt 1 - 2
- Mg²⁺ = 0 - 3: bevorzugt 0,5 - 1,5
- Cl⁻ = 90,5 - 121: bevorzugt 105 - 115
- Na⁺ = 128 - 145: bevorzugt 135 - 140
- K⁺ = 0 - 4: bevorzugt 1 - 3
- HCO₃ = 25 - 40: bevorzugt 28 - 35

Wie festgestellt, wird durch die Säure aus dem Hydrogencarbonat ca. 2 - 5 mmol/l CO₂ freigesetzt, das in dem Gemisch physikalisch gelöst ist und mit gasförmigem CO₂ im Gleichgewicht steht. Insofern kann die fertige Lösung einen Partialdruck pCO₂ von etwa 50 - 90 mm/Hg aufweisen.

Insgesamt ist festzustellen, daß die fertige Dialysierflüssigkeit im wesentlichen einen physiologischen Elektrolytgehalt aufweisen soll, dervon Fall zu Fall patientenspezifisch angepaßtsein kann. Es bestehen daher innerhalb dieses physiolgischen Bereichs bestimmte Formulierungsmöglichkeiten.

Für den Fall, daß die bicarbonathaltige Lösung osmotische Eigenschaften aufweisen soll, was bei dem Einsatz für die CAPD notwendig ist, weist sie ein osmotisch wirksames Agens in entsprechenden Mengen auf. Hierzu wird derzeit insbesondere Glucose eingesetzt. Im vorliegenden Fall enthält das saure Konzentrat ca. 26 - 90g Glucose/l, was bei der 1:1-Verdünnung zu einer Osmolarität der Lösung von etwa 350 - 550 mosm/l führt. Vorteilhafterweise hält man den pH-Wert des sauren Konzentrats, das auch die Glucose enthält, bei 5,5 - 6,2. Hierdurch wird beim Sterilisieren bei erhöhten Temperaturen (121°C) gewährleistet, daß eine Karamelisierung der Glucose vermieden wird. Dieser geringe Säuregrad des sauren Konzentrates verändert im übrigen kaum den pH-Wert der Mischung gegenüber dem pH-Wert des basischen Konzentrats (B), da die Pufferkapazität des Natriumbicarbonat-Puffers eine derartige geringe Menge an Protonen ohne weiteres wegpuffert.

Wie bereits vorstehend erwähnt, sind in den beiden Konzentraten (A) und (B) nur solche Ionen getrennt zu halten, die miteinander unter Bildung von schwerlöslichen Carbonaten ausgefällt werden können. Ansonsten sind nur praktische Erwägungen maßgebend, in welchem der Konzentrate die übrigen Bestandteile vorgelegt werden. Insofern werden also das Konzentrat (A) die Calcium- und Magnesiurnsalze und das Konzentrat (B) das Natriumhydrogencarbonat aufweisen. Ansonsten entscheiden andere Gesichtspunkte (beispielsweise der Säuregrad für die Karamelisierung der Glucose), in welchem der Konzentrate die übrigen Bestandteile, wie Kaliumchlorid, Natriumchlorid u.dgl., vorgelegt werden.

Das Beispiel erläutert die Erfindung.

### Beispiel

### Konzentrat (B)

Es werden 76 mmol/l Natriumhydrogencarbonat in 1 Liter Wasser eingewogen. Anschließend wird die erhaltene Lösung mit 1 n HCl auf einen pH-Wert von 7,35 - 7,4 eingestellt. Dabei wird die gesamte Lösung pyrogenfrei filtriert und danach bei 121°C sterilisiert, wobei - wie üblich - die in dem geschlossenen Gefäß auftretenden Überdrücke bzw. Partialdrücke kompensiert werden.

### Konzentrat (A)

Es werden
196 mmol/l NaCl,
3,0 mmol CaCl₂ x 2 H₂O,
1 mmol/l MgCl₂ x 6 H₂O,
1,66 mmol/l Glucosemonohydrat
eingewogen, wobei dieses Gemisch mit Wasser auf ein Volumen von 1 Liter aufgefüllt wird. Der pH-Wert wird mit 1 n HCl auf einen Wert von 5,5 (einige Tropfen) eingestellt.

Die so hergestellte Lösung wird ebenfalls pyrogenflei filtriert und danach, wie das Konzentrat (B), hitzesterilisiert.

Vorteilhafterweise werden beide Konzentrate vor dem Sterilisieren in einen 2-Kammerbeutel abgefüllt, dessen Kammern durch eine aufbrechbare Verbindungseinrichtung miteinander in Fluidverbindung gebracht werden können.

Dabei wird das Konzentrat (B) in der Kammer vorgelegt, die mit der Ausflußöffnung in Verbindug steht. Um die beiden Konzentrate zu vermischen, wird dann die aufzubrechende Verbindungseinrichtung aufgebrochen, wobei das Konzentrat (A) in das Bicarbonatkonzentrat (B) durch Druck auf den Beutel überführt wird. Die Durchmischung der beiden Konzentrate erfolgt dadurch, daß die fertigen Mischungen wechselweise von der einen Kammer in die andere Kammer und zurück gepumpt werden.

Danach ist die Bicarbonatiösung für die CAPD einsetzbar und weist folgende Zusammensetzung auf:
- Natrium 136: mmol/l
- Calcium 1,5: mmol/l
- Magnesium 0,5: mmol/l
- Glucose 0,83: mmol/l
- Hydrogencarbonat 38: mmol/l
- Chlorid 102: mmol/l
- pH-Wert: 7,3

Diese Lösung weist nach 6-stündigem Stehen keine Trübung durch Ausfällen von Calciumcarbonat auf und kann auf die übliche Weise für die CAPD eingesetzt werden.

## Patentansprüche

1. Zweikammerbeutelsystem zur Herstellung einer für die CAPD einsetzbaren Dialysierflüssigkeit, die folgende Zusammensetzung in mval/l aufweist:
Ca²⁺ = 0,5 - 5
Mg²⁺ = 0 - 3
Cl⁻ = 90,5 - 121
Na⁺ = 128 - 145
K⁺ = 0 - 4
HCO₃⁻ = 25 - 40,
wobei die eine Kammer des Beutelsystems ein saures, wenigstens Calciumionen enthaltendes Konzentrat und die andere Kammer ein basisches, wenigstens Bicarbonationen aufweisendes calciumionenfreies zweites Konzentrat aufweist, wobei dem basischen Konzentrat physologisch verträgliche Säure zugesetzt ist, und beide Konzentrate nach Beseitigung der Kammertrennmittel miteinander unter Bildung der Dialysierflüssigkeit vermischbar sind, dadurch gekennzeichnet, daß dem basischen Konzentrat soviel physiologisch verträgliche Säure zugesetzt ist, daß der pH-Wert unterhalb 7, 6, bei Raumtemperatur liegt.

2. Zweikammerbeutelsystem nach Anspruch 1, dadurch gekennzeichnet, daß dem basischen Konzentrat soviel physiologisch verträgliche Säure zugesetzt ist, daß der pH-Wert im Bereich von 7,2 - 7,4 liegt.

3. Zweikammerbeutelsystem nach Anspruch 1 oder 2, dadurch gekennzeichnet, daß das basische Konzentrat soviel Natriumbicarbonat aufweist, daß der Hydrogencarbonatgehalt der fertigen Dialysierflüssigkeit wenigstens 20 mmol/l beträgt.

4. Zweikammerbeutelsystem nach Anspruch 3, dadurch gekennzeichnet, daß der Hydrogencarbonatgehalt des basischen Konzentrates so groß ist, daß die fertige Dialysieflüssigkeit 25 - 40 mmol/l Bicarbonationen aufweist.

5. Zweikammerbeutelsystem nach einem der Ansprüche 1 bis 4, dadurch gekennzeichnet, daß der pH-Wert des basischen Konzentrates durch HCl eingestellt worden ist.

6. Zweikammerbeutelsystem nach einem der Ansprüche 1 bis 5, dadurch gekennzeichnet, daß die beiden Konzentrate in einem Mischungsverhältnis von 3:1 bis 1:3, insbesondere etwa 1:1, vorliegen.

## Claims

1. Two-chamber bag system for the preparation of a dialysis fluid which can be used for CAPD, having the following compositional ranges in mval/l:
Ca² = 0.5 - 5
Mg²⁺ = 0 - 3
Cl⁻ = 90.5 - 121
Na⁺ = 128 - 145
K⁺ = 0 - 4
HCO₃ = 25 - 40
in which one chamber of the bag system holds an acid concentrate containing, at least, calcium ions, and the other chamber holds a basic second concentrate, free from calcium ions and containing, at least, bicarbonate ions, where physiologically compatible acid is added to the basic concentrate, and where the two concentrates can be mixed together, following the breaking of the breakable connecting arrangement, with the formation of the dialysis fluid, distinguished by the fact that the physiologically compatible acid is added to the basic concentrate in an amount such that the pH value is below 7.6 at room temperature.

2. Two-chamber bag system as Claim 1, distinguished by the fact that the physiologically compatible acid is added to the basic concentrate in an amount such that the pH value is in the range of 7.2 to 7.4.

3. Two-chamber bag system as Claim 1 or 2, distinguished by the fact that the basic concentrate contains sodium bicarbonate in an amount such that the hydrogen carbonate content of the finished dialysis fluid is at least 20 mmol/l.

4. Two-chamber bag system as Claim 3, distinguished by the fact that the hydrogen carbonate content of the basic concentrate is so great that the finished dialysis fluid has between 25 and 40 mmol/l of bicarbonate ions.

5. Two-chamber bag system as Claims 1 to 4, distinguished by the fact that the pH value of the basic concentrate has been adjusted using HCl.

6. Two-chamber bag system as one of Claims 1 to 5, distinguished by the fact that the two concentrates are mixed with each other in a ratio of 3:1 to 1:3, and in particular of approximately 1:1.

## Revendications

1. Système de poche avec deux chambres pour la préparation d'un liquide de dialyse se prêtant à la CAPD, qui présente la composition suivante en mval/l :
Ca²⁺ = 0,5 - 5
Mg²⁺ = 0 - 3
Cl⁻ = 90,5 - 121
Na⁺ = 128 - 145
K⁺ = 0 - 4
HCO₃⁻ = 25 - 40
dans lequel l'une des chambres du système de poche présente un concentré contenant au moins des ions calcium et l'autre chambre, un deuxième concentré basique, exempt d'ions calcium, présentant au moins des ions bicarbonate, dans lequel de l'acide physiologiquement acceptable est ajouté au concentré basique, et dans lequel les deux concentrés sont miscibles l'un avec l'autre après suppression du moyen de séparation des chambres avec formation du liquide de dialyse, caractérisé en ce que suffisamment d'acide physiologiquement acceptable est ajouté au concentré basique pour que la valeur du pH soit inférieure à 7,6 à température ambiante.

2. Système de poche avec deux chambres suivant la revendication 1, caractérisé en ce que suffisamment d'acide physiologiquement acceptable est ajouté au concentré basique pour que la valeur du pH soit dans l'intervalle de 7,2 - 7,4.

3. Système de poche avec deux chambres suivant la revendication 1 ou 2, caractérisé en ce que le concentré basique présente suffisamment de bicarbonate de sodium pour que la teneur en hydrogénocarbonate du liquide de dialyse prêt à l'usage soit d'au moins 20 mmoles/l.

4. Système de poche avec deux chambres suivant la revendication 3, caractérisé en ce que la teneur en hydrogénocarbonate du concentré basique est suffisante pour que le liquide de dialyse prêt à l'usage présente 25 - 40 mmoles/l d'ions bicarbonate.

5. Système de poche avec deux chambres suivant l'une quelconque des revendications 1 à 4, caractérisé en ce que la valeur du pH du concentré basique a été ajustée au moyen de HCl.

6. Système de poche avec deux chambres suivant l'une des revendications 1 à 5, caractérisé en ce que les deux concentrés se présentent dans un rapport de mélange de 3:1 à 1:3, en particulier environ 1:1.
